Europäisches Patentamt

European Patent Office

Office européen des brevets

(11) Veröffentlichungsnummer: **0 446 751 A1**

# (12) EUROPÄISCHE PATENTANMELDUNG

(21) Anmeldenummer: 91103213.4

(22) Anmeldetag: 04.03.91

(51) Int. Cl.5: **C07D 401/12,** C07D 409/12,
C07D 211/56, C07D 233/64,
C07D 265/28, C07C 255/26,
C07C 255/24, C07C 237/04,
C07C 237/22, C07C 227/40,
C07C 317/28

(30) Priorität: 16.03.90 DE 4008403

(43) Veröffentlichungstag der Anmeldung:
18.09.91 Patentblatt 91/38

(84) Benannte Vertragsstaaten:
AT BE CH DE DK ES FR GB GR IT LI LU NL SE

(71) Anmelder: MERCK PATENT GESELLSCHAFT
MIT BESCHRÄNKTER HAFTUNG
Frankfurter Strasse 250
W-6100 Darmstadt(DE)

(72) Erfinder: Raddatz, Peter. Dr.
Akazienweg 8 A
W-6104 Seeheim(DE)
Erfinder: Schmitges, Claus, J.
Karolinger Strasse 5
W-6114 Gross-Umstadt(DE)
Erfinder: Minck, Klaus-Otto, Dr.
Büchestrasse 8
W-6105 Ober-Ramstadt(DE)

(54) Glykolsäurederivate.

(57) Neue Glykolsäurederivate der Formel I

$$X-O-CR^1R^2-CO-Y-NR^3-CHR^4-CR^5-CH_2-cR^6R^7-Z \qquad I$$

worin $R^1$ bis $R^7$, X, Y und Z die in Patentanspruch 1
angegebenen Bedeutungen haben,
sowie ihre Salze hemmen die Aktivität des menschlichen Plasmarenins.

Glykolsäurederivate der Formel I

$$X-O-CR^1R^2-CO-Y-NR^3-CHR^4-CR^5-CH_2-CR^6R^7-Z \quad I$$

worin

X

$R^8$, $R^8-O-C_mH_{2m}-CO-$, $R^8-C_mH_{2m}-O-CO-$, $R^8-C_mH_{2m}-CO-$, $R^8-SO_2-$, $R^9R^{10}N-C_mH_{2m}-CO-$, $R^{11}-NH-C(=NH)-NH-C_mH_{2m}-CO-$, $R^9OOC-C_mH_{2m}-CO-$, $R^9O_3S-C_mH_{2m}-CO-$, $R^9-O-(CH_2CH_2O)_nC_mH_{2m}-CO-$ oder $An^-N^+A_3-C_mH_{2m}-CO-$,

Y

0 oder 1 Aminosäurerest ausgewählt aus der Gruppe bestehend aus Abu, Ada, Ala, βAla, Arg, Asn, Asp, Bia, Cal, Cys, S-A-Cys, Dab, Gln, Glu, Gly, His, N(im)-A-His, Hph, Ile, Leu, tert.-Leu, Lys, Mal, Met, αNal, βNal, Nbg, Nle, Nva, Orn, Phe, Pia, Pro, Pya, Ser, Thr, Tia, Tic, Trp, Tyr und Val,

Z

$-CN$, $-CH_2-NR^{12}R^{13}$, $-CH_2-NR^{12}-SO_2R^{14}$, $-CH_2-NR^{12}-COR^{14}$, $-CH_2-NR^{12}-CO-NH-R^{14}$, oder $-CH_2-NR^{12}-CS-NH-R^{14}$

$R^1, R^3, R^6, R^7, R^9, R^{10}, R^{12}$ und $R^{13}$

jeweils H oder A,

$R^2, R^4, R^8$ und $R^{14}$

jeweils H, A, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^5$

(H, OH), (H, NH$_2$) oder = O,

$R^{11}$

H, A oder CN,

$R^9R^{10}N$ und $NR^{12}R^{13}$

auch jeweils eine unsubstituierte oder eine durch A, OH, NH$_2$, NHA, NA$_2$, NHAc, NH-CO-C$_x$H$_{2x}$-O-R$^{15}$, NH-CO-O-C$_x$H$_{2x}$-R$^{15}$, Hydroxyalkyl, COOH, COOA, CONH$_2$, Aminoalkyl, HAN-alkyl, A$_2$N-alkyl, A$_3$N$^⊕$alkyl An$^⊖$, NH-CO-NH$_2$, NH-CO-NHA, Guanidinyl oder Guanidinyl-alkyl substituierte Pyrrolidino-, Piperidino-, Morpholino- oder Piperazino-gruppe,

$R^{15}$

A oder Ar-alkyl,

m, n und x

jeweils 0, 1, 2, 3, 4, 5, 6, 7, 8, 9 oder 10,

Ar

unsubstituiertes oder ein-oder mehrfach durch A, OA, Hal, CF$_3$, OH, NO$_2$, Hydroxyalkyl, NH$_2$, NHA NA$_2$, NHAc, NH-SO$_2$-A, SA, SO-A, SO$_2$-A, SO$_2$NH$_2$, SO$_2$NHA, COOH, COOA, CONH$_2$, CN, Amino-alkyl, HAN-alkyl, A$_2$N-alkyl, A$_3$N$^⊕$ -alkyl An$^⊖$ und/oder Guanidinyl-alkyl substituiertes Phenyl oder unsubstituiertes Naphthyl,

Het

einen gesättigten oder ungesättigten 5-oder 6-gliedrigen heterocyclischen Rest mit 1-4 N-, O- und/oder S-Atomen, der mit einem Benzolring kondensiert und/oder ein-oder mehrfach durch A, OA, Hal, CF$_3$, OH, NO$_2$, Carbonylsauerstoff, NH$_2$, NHA, NA$_2$, NHAc, NH-COOA, NHCOOAr, NHCOOCH$_2$Ar, NH-SO$_2$-A, SA, SO-A, SO$_2$-A, SO$_2$NH$_2$, SO$_2$NHA, COOH, COOA, CONH$_2$, CN, Ar, Ar-alkyl, Ar-alkenyl, Hydroxy-alkyl, Amino-alkyl, HAN-alkyl, A$_2$N-alkyl und/oder A$_3$N$^⊕$-alkyl An$^⊖$ substituiert sein kann und/oder dessen N- und/oder S-Heteroatome auch oxydiert sein können,

Hal

F, Cl, Br oder J,

Ac

A-CO-, Ar-CO-, Ar-alkyl-CO- oder A-NH-CO-,

An$^⊖$

ein Anion, das auch fehlen kann, wenn stattdessen eine in der Verbindung der Formel I enthaltene Carboxygruppe in Form eines Carboxylatanions vorliegt,

-alkyl-

eine Alkylengruppe mit 1-8 C-Atomen und

A

Alkyl mit 1-8 C-Atomen bedeuten,

worin ferner an stelle einer oder mehrerer -NH-CO-Gruppen auch eine oder mehrere -NA-CO-Gruppen stehen können,

sowie deren Salze.

Ähnliche Verbindungen sind aus der EP-A-249096 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000mal so hohe Konzentrationen dieser Verbindungen notwendig wie für die Renin-Hemmung. Die Wirkungen der Verbindungen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden. Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste -NR'-R"-CO-, in der Regel -NH-CHR-CO- (worin R, R' und R" die für jede Aminosäure bekannte spezifische Bedeutung haben) folgender Aminosäuren:

| | |
|---|---|
| Abu | 2-Aminobuttersäure |
| Ada | 3-(1-Adamantyl)-alanin |
| Ala | Alanin |
| βAla | β-Alanin |
| Arg | Arginin |
| Asn | Asparagin |
| Asp | Asparaginsäure |
| Bia | 3-(2-Benzimidazolyl)-alanin |
| Cal | 3-Cyclohexylalanin |
| Cys | Cystein |
| S-A-Cys | S-Alkyl-cystein |
| S-Me-Cys | S-Methyl-cystein |
| Dab | 2,4-Diaminobuttersäure |
| Gln | Glutamin |
| Glu | Glutaminsäure |
| Gly | Glycin |
| His | Histidin |
| N(im)-A-His | in 1- oder 3-Stellung des Imidazolrings durch A substituiertes Histidin |
| Hph | Homophenylalanin (2-Amino-4-phenyl-buttersäure) |
| Ile | Isoleucin |
| Leu | Leucin |
| tert.-Leu | tert.-Leucin |
| Lys | Lysin |
| Mal | 3-(p-Methoxyphenyl)-alanin |
| Met | Methionin |
| αNal | 3-(α-Naphthyl)-alanin |
| βNal | 3-(β-Naphthyl)-alanin |
| Nbg | 2-Norbornyl-glycin |
| Nle | Norleucin |
| N-Me-His | N-Methyl-histidin |
| N-Me-Phe | N-Methyl-phenylalanin |
| Nva | Norvalin |

| | |
|---|---|
| Orn | Ornithin |
| Phe | Phenylalanin |
| Pia | 3-(Piperidyl)-alanin [z.B. 2-Pia = 3-(2-Piperidyl)-alanin] |
| Pro | Prolin |
| Pya | 3-(Pyridyl)-alanin [z.B. 3-Pya = 3-(3-Pyridyl)-alanin] |
| Ser | Serin |
| Thr | Threonin |
| Tia | 3-(Thienyl)-alanin [z.B. 2-Tia = 3-(2-Thienyl)-alanin] |
| Tic | 1,2,3, 4-Tetrahydroisochinolin-1-carbonsäure |
| Trp | Tryptophan |
| Tyr | Tyrosin |
| Val | Valin. |

Ferner bedeutet nachstehend:

| | |
|---|---|
| BOC | tert.-Butoxycarbonyl |
| BOM | Benzyloxymethyl |
| imi-BOM | Benzyloxymethyl in 1-Stellung des Imidazolrings |
| CBZ | Benzyloxycarbonyl |
| DCCI | Dicyclohexylcarbodiimid |
| DMF | Dimethylformamid |
| DNP | 2,4-Dinitrophenyl |
| imi-DNP | 2,4-Dinitrophenyl in 1-Stellung des Imidazolrings |
| ETOC | Ethoxycarbonyl |
| FMOC | 9-Fluorenylmethoxycarbonyl |
| HOBt | 1-Hydroxybenzotriazol |
| IPOC | Isopropoxycarbonyl |
| Pla | den Rest der Phenylmilchsäure -O-CH(CH$_2$C$_6$H$_5$)-CO-(S-Form) |
| POA | Phenoxyacetyl |
| THF | Tetrahydrofuran. |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Glykolsäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

X-G$^1$-OH    II

worin

G$^1$    (a) fehlt,

(b) -O-CR$^1$R$^2$-CO-,

(c) -O-CR$^1$R$^2$-CO-Y- bedeutet

oder eines ihrer reaktionsfähigen Derivate mit einer Verbindung der Formel III

H-G$^2$-NR$^3$-CH$^4$-CR$^5$-CH$_2$-CR$^6$R$^7$-Z    III

worin

G$^2$    (a) -O-CR$^1$R$^2$-CO-Y-,

(b) -Y- bedeutet,

(c) fehlt,

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder zur Herstellung einer Verbindung der Formel I, R$^5$ = (H, OH) oder (H, NH$_2$), ein Aminoketosäurederivat der Formel I, R$^5$ = O, reduziert oder reduktiv aminiert und/oder eine Verbindung der Formel I durch Behandeln mit einer Säure in eines ihrer Salze überführt.

Vor- und nachstehend haben die Reste bzw. Parameter R$^1$ bis R$^{15}$, X, Y, Z, m, n, x, Ar, Het, Hal, Ac, An, A, G$^1$ und G$^2$ die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl,  1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Dementsprechend bedeutet Cycloalkylalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo-[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m- oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m- oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, o-, m- oder p-Dimethylaminomethylbenzyl, o-, m- oder p-Guanidinomethylbenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten:
2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxy-phenyl-1H-2-pyridon-3-yl, 2-Methyl-3-hydroxy-4-hydroxy-methyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-methyl-4-pyrimidinyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5- oder -6-benzimidazolyl, 1-Ethyl-5- oder -6- benzimidazolyl, 3-, 4-, 5-, 6-, 7- oder 8-Hydroxy-2-chinolyl, 2-Oxo-pyrrolidino, 2-Oxo-piperidino, 2,5-Dioxopyrrolidino, 3-Benzyl-2,5-dioxopyrrolidino.

X ist allgemein vorzugsweise $R^8$, $R^8$-$C_mH_{2m}$-O-CO-, $R^9R^{10}$N-$C_mH_{2m}$-CO-, insbesondere 4-BOC-amino-piperidinocarbonyl oder 4-Amino-piperidinocarbonyl.

Die Gruppe Y besteht vorzugsweise aus einem der angegebenen Aminosäurereste; sie kann jedoch auch fehlen. Vorzugsweise bedeutet Y His, Leu oder S-Me-Cys, ferner bevorzugt βAla, Asn, N-Me-His, Met, Nle, Nva, Pya (besonders 3-Pya), Tia oder Val.

Z ist allgemein vorzugsweise -CN, -$CH_2$-$NR^{12}$-$SO_2R^{14}$ oder -$CH_2$-$NR^{12}$-CO-$NHR^{14}$, ferner bevorzugt -$CH_2$-$NR^{12}$-CS-$NHR^{14}$ oder -$CH_2$-$NR^{12}$-$COR^{14}$.

$R^1$, $R^3$, $R^5$, $R^7$, $R^9$, $R^{10}$, $R^{12}$ und $R^{13}$ bedeuten jeweils bevorzugt H, ferner bevorzugt Methyl; $R^6$ ist bevorzugt auch Isopropyl oder Isobutyl. $R^9R^{10}$N ist bevorzugt auch Pyrrolidino, Piperidino, Morpholino, Amino-piperidino wie 4-Aminopiperidino, Alkylaminopiperidino wie 4-Methylaminopiperidino, Dialkylaminopiperidino wie 4-Dimethylaminopiperidino oder BOC-amino-piperidino wie 4-BOC-aminopiperidino.

$R^2$ ist vorzugsweise Ar-alkyl, insbesondere Benzyl oder p-Methoxybenzyl; ferner bevorzugt A, insbesondere n-Butyl oder Isobutyl; Cycloalkyl-alkyl, insbesondere Cyclohexylmethyl; Het-alkyl, insbesondere 2-Thienylmethyl. Die Gruppe -O-$CR^1R^2$-CO- bedeutet bevorzugt den Rest Pla.

$R^4$ ist vorzugsweise Cycloalkylalkyl, insbesondere Cyclohexylmethyl, ferner bevorzugt Alkyl, insbesondere n-Butyl oder Isobutyl; Ar-alkyl, insbesondere Benzyl oder p-Methoxybenzyl; Het-alkyl, z. B. 2-Thienylmethyl; Cycloalkyl, insbesondere Cyclohexyl.

$R^5$ ist vorzugsweise (H, OH).

$R^8$ ist vorzugssweise A, insbesondere Methyl, Ethyl, Isopropyl oder tert.-Butyl; oder Ar, insbesondere Phenyl, 1- oder 2-Naphthyl.

$R^{11}$ ist vorzugsweise H, Methyl oder CN.

$R^{14}$ ist vorzugsweise A, insbesondere Methyl, Ethyl, Propyl, Isopropyl, Butyl, Isobutyl, sek.-Butyl, tert.-Butyl, Pentyl oder Isopentyl; H; Cycloalkylalkyl, insbesondere Cyclohexylmethyl; Ar-alkyl, insbesondere Benzyl; Ar, insbesondere Phenyl oder Aminophenyl wie p-Aminophenyl.

$R^{15}$ ist vorzugsweise A mit 1-4 C-Atomen, insbesondere Isopropyl oder tert.-Butyl; oder Ar-alkyl, insbesondere Benzyl.

Der Parameter m ist vorzugsweise 1, 2, 3, 4 oder 5; n ist vorzugsweise 1; x ist vorzugsweise 1 oder 2.

$C_mH_{2m}$ und $C_xH_{2x}$ sind vorzugsweise geradkettig, bedeuten also vorzugsweise -$(CH_2)_m$- oder -$(CH_2)_x$-.

Dementsprechend bedeutet die Gruppe X im einzelnen vorzugsweise A, z. B. Isopropyl oder Isobutyl; Ar-alkyl, z. B. Benzyl; $R^8$-$(CH_2)_m$-O-CO-,

z. B. BOC oder CBZ; $R^9R^{10}N$-$(CH_2)_m$-CO-, vor allem $H_2N$-$C_mH_{2m}$-CO- wie Aminocarbonyl, Aminoacetyl (H-Gly-), 3-Aminopropionyl (H-βAla-), 4-Aminobutyryl, 5-Aminopentanoyl, 6-Aminohexanoyl, 7-Aminoheptanoyl, 8-Aminooctanoyl, 9-Aminononanoyl, 10-Aminodecanoyl,11-Aminoundecanoyl, aber auch z.B. 2-Amino-propionyl (Ala), 2-Amino-2-methylpropionyl; ANH-$C_mH_{2m}$-CO- wie Methylaminocarbonyl, Methylaminoacetyl (Sarcosyl), 3-Methylaminopropionyl, 4-Methylaminobutyryl, 5-Methylaminopentanoyl, 6-Methylaminohexanoyl, 6-Ethylaminohexanoyl, 7-Methylaminoheptanoyl, 8-Methylaminooctanoyl, 9-Methylaminononanoyl, 10-Methylaminodecanoyl, 11-Methylaminoundecanoyl; $A_2N$-$C_mH_{2m}$-CO-wie Dimethylaminocarbonyl, Dimethylaminoacetyl, 3-Dimethylaminopropionyl, 4-Dimethylaminobutyryl, 5-Dimethylaminopentanoyl, 6-Dimethylaminohexanoyl, 6-Diethylaminohexanoyl, 7-Dimethylaminoheptanoyl, 8-Dimethylaminooctanoyl, 9-Dimethylaminononanoyl, 10-Dimethylaminodecanoyl, 11-Dimethylaminoundecanoyl; Pyrrolidino-$C_mH_{2m}$-CO- wie Pyrrolidinocarbonyl, Pyrrolidinoacetyl, 3-Pyrrolidinopropionyl, 4-Pyrrolidino-butyryl, 5-Pyrrolidino-pentanoyl, 6-Pyrrolidino-hexanoyl, 7-Pyrrolidino-heptanoyl, 8-Pyrrolidino-octanoyl, 9-Pyrrolidino-nonanoyl, 10-Pyrrolidinodecanoyl; Piperidino-$C_mH_{2m}$-CO- wie Piperidinocarbonyl, Piperidinoacetyl, 3-Piperidino-propionyl, 4-Piperidinobutyryl, 5-Piperidino-pentanoyl, 6-Piperidino-hexanoyl, 7-Piperidinoheptanoyl, 8-Piperidino-octanoyl, 9-Piperidino-nonanoyl, 10-Piperidino-decanoyl; Morpholino-$C_mH_{2m}$-CO-wie Morpholinocarbonyl, Morpholinoacetyl, 3-Morpholino-propionyl, 4-Morpholino-butyryl, 5-Morpholinopentanoyl, 6-Morpholino-hexanoyl, 7-Morpholino-heptanoyl, 8-Morpholino-octanoyl, 9-Morpholino-nonanoyl, 10-Morpholino-decanoyl;4-Amino-piperidino-$C_mH_{2m}$-CO- wie 4-Aminopiperidino-carbonyl, 4-Amino-piperidino-acetyl, 3-(4-Amino-piperidino)-propionyl, 4-(4-Amino-piperidino)-butyryl, 5-(4-Amino-piperidino)-pentanoyl, 6-(4-Aminopiperidino)-hexanoyl, 7-(4-Amino-piperidino)-heptanoyl, 8-(4-Amino-piperidino)-octanoyl, 9-(4-Amino-piperidino)-nonanoyl, 10-(4-Amino-piperidino)-decanoyl; 4-Dialkylamino-piperidino-$C_mH_{2m}$-CO-wie 4-Dimethylamino-piperidinocarbonyl, 4-Dimethylamino-piperidino-acetyl; 4-Guanidinopiperidino-$C_mH_{2m}$-CO- wie 4-Guanidinopiperidino-carbonyl, 4-Guanidino-piperidinoacetyl; 4-Carboxy-piperidino-$C_mH_{2m}$-CO-wie 4-Carboxy-piperidino-carbonyl, 4-Carboxy-piperidino-acetyl; 4-Alkoxycarbonyl-piperidino-$C_mH_{2m}$-CO- wie 4-Methoxycarbonyl-piperidino-carbonyl, 4-Ethoxycarbonylpiperidino-carbonyl,4-Methoxycarbonyl-piperidino-acetyl, 4-Ethoxycarbonyl-piperidino-acetyl; 4-AcNH-piperidino-$C_mH_{2m}$-CO- wie 4-Acetamido-piperidinocarbonyl, 4-Acetamido-piperidino-acetyl; $H_2N$-C-( = NH)-NH-$C_mH_{2m}$-CO- wie Guanidinoacetyl, 3-Guanidino-propionyl, 4-Guanidinobutyryl, 5-Guanidino-pentanoyl, 6-Guanidino-hexanoyl, 7-Guanidino-heptanoyl, 8-Guanidino-octanoyl; NC-NH-C( = NH)-NH-$C_mH_{2m}$-CO- wie N'-Cyanguanidinoacetyl, 3-(N'-Cyanguanidino)-propionyl, 4-(N'-Cyanguanidino)-butyryl, 5-(N'-Cyanguanidino)-pentanoyl, 6-(N'-Cyanguanidino)-hexanoyl, 7-(N'-Cyanguanidino)-heptanoyl, 8-(N'-Cyanguanidino)-octanoyl; HOOC-$C_mH_{2m}$-CO- wie Malonyl, Succinyl, Glutaryl, Adipyl, 6-Carboxyhexanoyl, 7-Carboxyheptanoyl, 8-Carboxyoctanoyl, 9-Carboxynonanoyl, 10-Carboxydecanoyl, 11-Carboxyundecanoyl; AOOC-$C_mH_{2m}$-CO- wie Methoxycarbonyl-acetyl, 3-Methoxycarbonyl-propionyl, 4-Methoxycarbonylbutyryl, 5-Methoxycarbonyl-pentanoyl, 6-Methoxycarbonylhexanoyl, 7-Methoxycarbonyl-heptanoyl, 8-Methoxycarbonyloctanoyl, 9-Methoxycarbonyl-nonanoyl, 10-Methoxycarbonyl-decanoyl, Ethoxycarbonyl-acetyl, 3-Ethoxycarbonyl-propionyl, 4-Ethoxycarbonyl-butyryl, 5-Ethoxycarbonyl-pentanoyl, 6-Ethoxycarbonyl-hexanoyl, 7-Ethoxycarbonyl-heptanoyl, 8-Ethoxycarbonyl-octanoyl, 9-Ethoxycarbonyl-nonanoyl, 10-Ethoxycarbonyl-decanoyl; H-$SO_3$-$C_mH_{2m}$-CO- wie Sulfo-acetyl, 3-Sulfo-propionyl, 4-Sulfobutyryl, 5-Sulfo-pentanoyl, 6-Sulfo-hexanoyl, 7-Sulfoheptanoyl, 8-Sulfo-octanoyl, 9-Sulfo-nonanoyl, 10-Sulfodecanoyl; A-$SO_3$-$C_mH_{2m}$-CO- wie Methoxysulfonylacetyl, 3-Methoxysulfonyl-propionyl, 4-Methoxysulfonyl-butyryl, 5-Methoxysulfonyl-pentanoyl, 6-Methoxysulfonyl-hexanoyl, 7-Methoxysulfonyl-heptanoyl, 8-Methoxysulfonyl-octanoyl, 9-Methoxysulfonyl-nonanoyl, 10-Methoxysulfonyl-decanoyl, Ethoxysulfonyl-acetyl, 3-Ethoxysulfonyl-propionyl, 4-Ethoxysulfonyl-butyryl, 5-Ethoxysulfonyl-pentanoyl, 6-Ethoxysulfonylhexanoyl, 7-Ethoxysulfonyl-heptanoyl, 8-Ethoxysulfonyl-octanoyl, 9-Ethoxysulfonyl-nonanoyl, 10-Ethoxysulfonyl-decanoyl; $R^8$-$C_mH_{2m}$-O-CO-, vor allem A-O-CO- wie ETOC, IPOC, BOC sowie Ar-$C_mH_{2m}$-O-CO- wie CBZ; $R^8$-$C_mH_{2m}$-CO- wie 3,3-Dimethylbutyryl.

Die Gruppe Z bedeutet dementsprechend im einzelnen vorzugsweise -CN; -$CH_2$NH-$SO_2R^{14}$, vor allem -$CH_2$-NH-$SO_2$-A wie Methan-, Ethan-, Propan-, 2-Methylpropan-, Butan-sulfonamidomethyl, ferner z.B. Phenylmethan-, Cyclohexylmethan-sulfonamidomethyl; -$CH_2$-NH-CO-NH-$R^{14}$-, vor allem -$CH_2$-NH-CO-NH-A wie N'-Methyl-ureido-methyl, N'-Ethyl-ureido-methyl, N'-Propyl-ureido-methyl, N'-Isopropyl-ureido-methyl, N'-Butyl-ureidomethyl, N'-Isopentyl-ureido-methyl, ferner -$CH_2$-NH-CO-NH-Ar wie N'-Phenyl-ureido-methyl, N'-p-Aminophenyl-ureido-methyl; -$CH_2$-NH-CS-NH-$R^{14}$, vor allem -$CH_2$-NH-CS-NH-A wie N'-Methyl-thioureido-methyl;-$CH_2$-NH-CO$R^{14}$, vor allem -$CH_2$-NH-CO-Awie Acetamidomethyl, Propionamidomethyl, Butyramidomethyl, Isovaleramidomethyl.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen. Falls $R^4$ von H verschieden ist und/oder $R^5$ (H, OH) oder (H, $NH_2$) bedeutet, sind die 4S-Hydroxy-, 4S-Amino-, 5S-Amino-, 4S-Hydroxy-5S-amino- bzw. 4S,5S-Diamino-Enantiomeren bevorzugt (wobei dem C-Atom, das den Rest $R^5$ trägt, die 4-Stellung, dem, das die Reste $X$-$O$-$CR^1R^2$-$CO$-$Y$-$NR^3$ und $R^4$ trägt, die 5-Stellung zugeordnet ist).

Die oben erwähnten Cycloalkyl- und Phenylgruppen sind vorzugsweise unsubstituiert oder tragen vorzugsweise 1 bis 3, insbesondere 1 oder 2 Substituenten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden:

| | |
|---|---|
| Ia | $R^8$-$O$-$CR^1R^2$-$CO$-$Y$-$NR^3$-$CHR^4$-$CR^5$-$CH_2$-$CR^6R^7$-$Z$; |
| Ib | $R^8$-$O$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| Ic | $R^8$-$C_mH_{2m}$-$O$-$CO$-$O$-$CR^1R^2$-$CO$-$Y$-$NR^3$-$CHR^4$-$CR^5$-$CH_2$-$CR^6R^7$-$Z$; |
| Id | $R^8$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| Ie | $R^9R^{10}N$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| If | $R^{11}$-$NH$-$C(=NH)$-$NH$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| Ig | $R^9OOC$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| Ih | $R^9O_3S$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| Ii | $R^9$-$O$-$(CH_2CH_2O)_n$-$C_mH_{2m}$-$CO$-$O$-$CR^1R^2$-$CO$-$Y$-$NR^3$-$CHR^4$-$CR^5$-$CH_2$-$CR^6R^7$-$Z$; |
| Ij | $R^9R^{10}N$-$CO$-$O$-$CR^1R^2$-$CO$-$Y$-$NR^3$-$CHR^4$-$CR^5$-$CH_2$-$CR^6R^7$-$Z$; |
| Ik | 4-Aminopiperidinocarbonyl-$O$-$CR^1R^2$-$CO$-$Y$-$NR^3$-$CHR^4$-$CR^5$-$CH_2$-$CR^6R^7$-$Z$. |

Insbesondere bevorzugt sind Verbindungen der Teilformeln:

(a) Iaa bis Ika, die den Formeln Ia bis Ik entsprechen, worin jedoch zusätzlich
-$O$-$CR^1R^2$-$CO$- Pla bedeutet;

(b) Iab bis Ikb sowie Iaab bis Ikab, die den Formeln Ia bis Ik sowie Iaa bis Ika entsprechen, worin jedoch zusätzlich
Y     His, Leu oder S-Me-Cys bedeutet;
(c) Iac bis Ikc, Iaac bis Ikac, Iabc bis Ikbc sowie Iaabc bis Ikabc, die den Formeln Ia bis Ik, Iaa bis Ika, Iab bis Ikb sowie Iaab bis Ikab entsprechen, worin jedoch zusätzlich $R^4$ Cyclohexylmethyl bedeutet.

Insbesondere sind bevorzugt Verbindungen der Teilformeln:

$I^*$ sowie $Ia^*$ bis $Ik^*$, die den Formeln I sowie Ia bis Ik sowie solche Verbindungen, die den anderen vorstehend genannten Teilformeln entsprechen, worin jedoch zusätzlich
$R^5$     (H, OH) bedeutet;

I' sowie Ia' bis Ik', die den Formeln I sowie Ia bis Ik sowie solche Verbindungen, die den anderen vorstehend genannten Teilformeln entsprechen, worin jedoch zusätzlich
Z     - $CH_2$-$NHCONH$-A oder -$CH_2$-$NH$-$SO_2$-A bedeutet.

Eine besonders bevorzugte Gruppe von Verbindungen entspricht der Formel I,
worin
X 4-BOC-aminopiperidinocarbonyl oder 4-Aminopiperidinocarbonyl,

| | |
|---|---|
| $R^1$ $R^3$ und $R^7$ | jeweils H, |
| $R^2$ | Benzyl, |
| $R^4$ | Cyclohexylmethyl, |
| $R^5$ | (H, OH), |
| $R^6$ | H oder A, |
| Z | -$CH_2NHSO_2A$, |
| | -$CH_2NHCONHR^{14}$     oder |
| | -$CH_2NHCOA$ und |
| $R^{14}$ | H oder A bedeuten. |

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B.

solche die der Formel I entsprechen, aber an Stelle einer His-Gruppe eine N(im)-R'-His-Gruppe (worin R' eine Aminoschutzgruppe bedeutet, z.B. BOM oder DNP) enthalten, oder solche der Formel X-O-CR$^1$R$^2$-CO-Y-NR$^3$-CHR$^4$-CH(NHR')-CH$_2$-CR$^6$R$^7$-Z.

Ferner sind Ausgangsstoffe bevorzugt, die an Stelle des H-Atoms einer Hydroxygruppe eine Hydroxyschutzgruppe tragen, z.B. solche der Formel X-O-CR$^1$R$^2$-CO-Y-NR$^3$-CHR$^4$-CHOR''-CH$_2$-CR$^6$R$^7$-Z, worin R'' eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Kondensation (Peptidsynthese) aus einer Carbonsäure- (Formel II) und einer Hydroxy- bzw. Aminokomponente (Formel III) erhalten werden. Als Carbonsäurekomponenten eignen sich z.B. solche der Teilformeln (a) X-OH, (b) X-O-CR$^1$R$^2$-COOH oder (c) X-O-CR$^1$R$^2$-CO-Y-OH, als Hydroxy- bzw. Aminokomponenten solche der Teilformeln (a) HO-CR$^1$R$^2$-CO-Y-NR$^3$-CHR$^4$-CR$^5$-CH$_2$-CR$^6$R$^7$-Z, (b) H-Y-NR$^3$-CHR$^4$-CR$^5$-CH$_2$-CR$^6$R$^7$-Z oder (c) H-NR$^3$-CHR$^4$-CR$^5$-CH$_2$-CR$^6$R$^7$-Z. Dabei arbeitet man zweckmäßig nach üblichen Methoden der Peptid-Synthese, wie sie z.B. in Houben-Weyl, 1.c., Band 15/II, Seiten 1-806 (1974) beschrieben sind; diese Methoden können auch auf die Kondensation gemäß (a), wobei eine Esterbindung entsteht, übertragen werden.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxy-carbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Kondensation und der Abspaltung von Schutzgruppen hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine R$^{15}$-C$_x$H$_{2x}$-O-CO-NH-, eine AcNH-, eine ArCH$_2$SO$_3$- oder eine AOOC-Gruppe enthält, in die entsprechende Verbindung der Formel I umgewandelt werden, die stattdessen eine H$_2$N-, eine HSO$_3$- oder eine HOOC-Gruppe enthält, zweckmäßig durch selektive Solvolyse nach einer der oben angegebenen Methoden. AOOC-Gruppen können z.B. mit NaOH oder KOH in Wasser-Dioxan bei Temperaturen zwischen 0 und 40°, vorzugsweise 10 und 30°, verseift werden.

Es ist auch möglich, eine Verbindung der Formel I, die eine freie primäre oder sekundäre Aminogruppe enthält, zu acylieren. So kann man insbesondere Verbindungen der Formel I, in denen Z eine CH$_2$-NH-R$^{12}$-Gruppe bedeutet, mit acylierenden Mitteln der Formeln R$^{14}$-SO$_2$Cl, R$^{14}$-COCl oder R$^{14}$-NCO umsetzen, zweckmäßig in Gegenwart eines inerten Lösungsmittels wie THF und/oder einer Base wie Pyridin oder Triethylamin bei Temperaturen zwischen -10 und +30°.

Weiterhin können Ketoverbindungen der Formel I (R$^5$ = O) zu Verbindungen der Formel I (R$^5$ = (H, OH)) reduziert werden, beispielsweise mit einem komplexen Metallhydrid wie NaBH$_4$, das nicht gleichzeitig die Peptid-Carbonylgruppen reduziert, in einem inerten Lösungsmittel wie Methanol bei Temperaturen zwischen etwa -10 und +30°.

Ketoverbindungen der Formel I (R$^5$ = O) können auch durch reduktive Aminierung in Verbindungen der Formel I (R$^5$ = H, NH$_2$) übergeführt werden. Man kann ein- oder mehrstufig reduktiv aminieren. So kann man z.B. die Ketoverbindung mit Ammoniumsalzen, z.B. Ammoniumacetat, und NaCNBH$_3$ behandeln, vorzugsweise in einem inerten Lösungsmittel, z.B. einem Alkohol wie Methanol, bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-249096 beschriebenen Verbindungen verabreicht, vorzugsweise in Dosierungen zwischen etwa 10 mg und 1 g, insbesondere zwischen 50 und 500 mg pro Dosierungseinheit. Die tägliche Dosierung liegt vorzugsweise zwischen etwa 0,2 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertension und Hyperaldosteronismus können wirksam behandelt werden durch Verabfolgung von Dosierungen zwischen insbesondere etwa 1 und 300, vorzugsweise zwischen 5 und 50 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. TFA = Trifluoracetat.

Beispiel 1

Man löst 1 g N-Ethyl-N'-[4S-hydroxy-5S-(2S-(4-tert.-butoxycarbonylaminopiperidinocarbonyloxy)-3-phenyl-propionyl-L-(N-(imi)-benzyloxymethyl-histidyl)-amino)-6-cyclohexyl-hexyl]-harnstoff [ = N-Ethyl-N'-[4S-hydroxy-5S-(4-BOC-amino-piperidinocarbonyl-Pla-(imi-BOM-His)-amino)-6-cyclohexyl-hexyl]-harnstoff, F. 99-100°; erhältlich durch Abspaltung der BOC-Gruppe aus N-Ethyl-N'-[4S-hydroxy-5S-(BOC-(imi-BOM-His)-amino)-6-cyclohexyl-hexyl]-harnstoff (F. 75-76°) und Kondensation mit 4-BOC-amino-piperidinocarbonyloxy-Pla-OHdas seinerseits erhältlich ist durch Reaktion von 2S-Hydroxy-3-phenylpropionsäuremethylester ("Pla-OMe") mit Phosgen in siedendem Toluol und Umsetzung des erhaltenen 2S-Chlorcarbonyloxy-3-phenyl-propionsäuremethylesters mit 4-BOC-amino-piperidin in THF in Gegenwart von Triethylamin] in 25 ml Ethanol, hydriert an 0,5 g 10 %ig Pd-C bei 20° und 1 bar bis zum Stillstand der

H$_2$-Aufnahme, filtriert, dampft ein und erhält nach chromatographischer Reinigung N-Ethyl-N'-[4S-hydroxy-5S-(4-BOC-amino-piperidinocarbonyl-Pla-His-amino)-6-cyclohexyl-hexyl]-harnstoff, F. 180° (Zers.).

Analog erhält man durch Hydrogenolyse der entsprechenden imi-BOM-His-Derivate:
N-[4S-Hydroxy-5S-(4-BOC-amino-piperidinocarbonyl-Pla-His-amino)-6-cyclohexyl-hexyl]-harnstoff, F. 196-197°
N-Isopropyl-N'-[4S-hydroxy-5S-(4-BOC-amino-piperidinocarbonyl-Pla-His-amino)-6-cyclohexyl-hexyl]-harnstoff, F. 176-177°
N-[2R-Methyl-4S-hydroxy-5S-(4-BOC-amino-piperidinocarbonyl-Pla-His-amino)-6-cyclohexyl-hexyl]-harnstoff
N-Ethyl-N'-[2R-methyl-4S-hydroxy-5S-(4-BOC-amino-piperidinocarbonyl-Pla-His-amino)-6-cyclohexyl-hexyl]-harnstoff
N-Isopropyl-N'-[2R-methyl-4S-hydroxy-5S-(4-BOC-amino-piperidinocarbonyl-Pla-His-amino)-6-cyclohexyl-hexyl]-harnstoff, F. 200-201° [aus N-Isopropyl-N'-(2R-methyl-4S-hydroxy-5S-BOC-(imi-BOM-His)-amino-6-cyclohexyl-hexyl)-harnstoff (F. 146-147°) über N-Isopropyl-N'-[2R-methyl-4S-hydroxy-5S-(4-BOC-amino-piperidinocarbonyl-Pla-(imi-BOM-His)-amino-6-cyclohexyl-hexyl)-harnstoff (F. 120-121°)]
N-[2S-Isopropyl-4S-hydroxy-5S-(4-BOC-amino-piperidinocarbonyl-Pla-His-amino)-6-cyclohexyl-hexyl]-harnstoft
N-Ethyl-N'-[2S-isopropyl-4S-hydroxy-5S-(4-BOC-amino-piperidinocarbonyl-Pla-His-amino)-6-cyclohexyl-hexyl]-harnstoff, [aus N-Ethyl-N'-(2S-isopropyl-4S-hydroxy-5S-BOC-amino-6-cyclohexyl-hexyl)-harnstoff (F. 144-145°) über N-Ethyl-N'-[2S-isopropyl-4S-hydroxy-5S-BOC-(imi-BOM-His)-amino-6-cyclohexyl-hexyl]-harnstoff (F. 75-76°) und N-Ethyl-N'-[2S-isopropyl-4S-hydroxy-5S-(4-BOC-amino-piperidinocarbonyl-Pla-(imi-BOM-His)-amino-6-cyclohexyl-hexyl]-harnstoff, F. 106-107°]
N-Isopropyl-N'-[2S-isopropyl-4S-hydroxy-5S-(4-BOC-amino-piperidinocarbonyl-Pla-His-amino)-6-cyclohexyl-hexyl]-harnstoff
N-Ethyl-N'-[4S-hydroxy-5S-(2S-(4-BOC-amino-piperidinocarbonyloxy)-4-methyl-butyryl-His-amino)-6-cyclohexylhexyl]-harnstoff
N-Ethyl-N'-[2S-isopropyl-45-hydroxy-5S-(2s-(4-Boc-amino-piperidinocarbonyloxy)-4-methyl-butyryl-His-amino)-6-cyclohexyl-hexyl]-harnstoff
N-Ethyl-N'-[4S-hydroxy-5S-(2S-(4-BOC-amino-piperidinocarbonyloxy)-hexanoyl-His-amino)-6-cyclohexyl-hexyl]-harnstoff
N-Ethyl-N'-[2S-isopropyl-4S-hydroxy-5S-(2S-(4-Boc-amino-piperidinocarbonyloxy)-hexanoyl-His-amino)-6-cyclohexyl-hexyl]-harnstoff
N-Ethyl-N'-[4S-hydroxy-5S-(2S-(4-BOC-amino-piperidinocarbonyloxy)-3-cyclohexyl-propionyl-His-amino)-6-cyclohexyl-hexyl]-harnstoff
N-Ethyl-N'-[2S-isopropyl-4S-hydroxy-5S-(2S-(4-BOC-amino-piperidinocarbonyloxy)-3-cyclohexyl-propionyl-His-amino)-6-cyclohexyl-hexyl]-harnstoff
N-Ethyl-N'-[4S-hydroxy-5S-(2S-(4-BOC-amino-piperidinocarbonyloxy)-3-p-methoxyphenylpropionyl-His-amino)-6-cyclohexyl-hexyl]-harnstoff
N-Ethyl-N'-[2S-isopropyl-4S-hydroxy-5S-(2S-(4-BOC-amino-piperidinocarbonyloxy)-3-p-methoxyphenyl-propionyl-His-amino)-6-cyclohexyl-hexyl]-harnstoff
N-Ethyl-N'-[4S-hydroxy-5S-(2S-(4-BOC-amino-piperidinocarbonyloxy)-3-(2-thienyl)-propionyl-His-amino)-6-cyclohexyl-hexyl]-harnstoff
N-Ethyl-N'-[2S-isopropyl-4S-hydroxy-5S-(2S-(4-BOC-amino-piperidinocarbonyloxy)-3-(2-thienyl)-propionyl-His-amino)-6-cyclohexyl-hexyl]-harnstoff
N-[4S-Hydroxy-5S-(4-BOC-amino-piperidinocarbonyl-Pla-His-amino)-6-cyclohexyl-hexyl]-propansulfonsäureamid, F. 164-165°
N-[2R-Methyl-4S-hydroxy-5S-(4-BOC-amino-piperidinocarbonyl-Pla-His-amino)-6-cyclohexyl-hexyl]-propansulfonsäureamid, F. 116-117° [aus N-(2R-Methyl-4S-hydroxy-5S-BOC-(imi-BOM-His)-amino-6-cyclohexyl-hexyl)-propansulfonsäureamid (F. 147-148°) über N-[2R-Methyl-4S-hydroxy-5S-(4-BOC-amino-piperidinocarbonyl)-Pla-(imi-BOM-His)-amino)-6-cyclohexyl-hexyl]-propansulfonsäureamid (F. 90-91°)]
N-[2S-Isopropyl-4S-hydroxy-5S-(4-BOC-amino-piperidinocarbonyl-Pla-His-amino)-6-cyclohexyl-hexyl]-propansulfonsäureamid, [aus N-(2S-Isopropyl-4S-hydroxy-5S-BOC-amino-6-cyclohexyl-hexyl)-propansulfonsäureamid (Öl) über N-(2S-Isopropyl-4S-hydroxy-5S-BOC-(imi-BOM-His)-amino-6-cyclohexyl-hexyl)-propansulfonsäureamid (F. 101-102°)]
N-[4S-Hydroxy-5S-(4-BOC-amino-piperidinocarbonyl-Pla-His-amino)-7-methyl-octyl]-propansulfonsäureamid
N-[2R-Methyl-4S-hydroxy-5S-(4-BOC-amino-piperidinocarbonyl-Pla-His-amino)-7-methyl-octyl]-propansulfonsäureamid
N-[2S-Isopropyl-4S-hydroxy-5S-(4-BOC-amino-piperidinocarbonyl-Pla-His-amino)-7-methyl-octyl]-propansulfonsäureamid
N-[4S-Hydroxy-5S-(4-BOC-amino-piperidinocarbonyl-Pla-His-amino)-6-phenyl-hexyl]-propansulfonsäureamid
N-[2R-Methyl-4S-hydroxy-5S-(4-BOC-amino-piperidinocarbonyl-Pla-His-amino)-6-phenyl-hexyl]-propansulfonsäureamid
N-[2S-Isopropyl-4S-hydroxy-5S-(4-BOC-amino-piperidinocarbonyl-Pla-His-amino)-6-phenyl-hexyl]-propansulfonsäureamid
N-[4S-Hydroxy-5S-(4-BOC-amino-piperidinocarbonyl-Pla-His-amino)-6-cyclohexyl-hexyl]-isovaleriansäureamid, F. 176-177° [aus N-3-(3-BOC-4-cyclohexylmethyl-2,2-dimethyl-5-oxazolidinyl)-propyl-isovaleriansäureamid (Öl) über N-[4S-Hydroxy-5-BOC-(imi-BOM-His)amino-6-cyclohexyl-hexyl]-isovaleriansäureamid (F. 135-136°) und N-(4S-Hydroxy-5S-(4-BOC-amino-piperidinocarbonyl-Pla-(imi-BOM-His)-amino)-6-cyclohexyl-hexyl]-isovaleriansäureamid (F. 93-94°)].

Beispiel 2

Ein Gemisch von 1011 mg N-[2R-Methyl-4S-hydroxy-5S-(2S-(4-BOC-amino-piperidinocarbonyloxy)-3-phenyl-propionyl-L-(N-(imi)-2,4-dinitrophenyl-histidyl)-amino)-6-cyclohexyl-hexyl]-propansulfonsäureamid [= N-[2R-Methyl-4S-hydroxy-5S-(4-BOC-amino-piperidinocarbonyl-Pla-(imi-DNP-His)-amino)-6-cyclohexyl-hexyl]-propansulfonsäureamid; erhältlich durch Kondensation von 4-BOC-amino-piperidinocarbonyl-Pla-OH mit N-[2R-Methyl-4S-hydroxy-5S-(H-(imi-DNP-His)-amino)-6-cyclohexyl-hexyl]-propansulfonsäureamid analog Beispiel 3], 2 g 2-Mercaptoethanol, 20 ml DMF und 20 ml Wasser wird unter Rühren bei 20° mit wässeriger $Na_2CO_3$-Lösung auf pH 8 eingestellt und noch 2 Std. bei 20° gerührt. Nach üblicher Aufarbeitung erhält man N-[2R-Methyl-4S-hydroxy-5S-(4-BOC-amino-piperidinocarboyl-Pla-His-amino)-6-cyclohexyl-hexyl]-propansulfonsäuremid, F. 116-117°.

Analog erhält man aus den entsprechenden imi-DNP-His-Derivaten die übrigen in Beispiel 1 angegebenen Verbindungen.

Beispiel 3

Analog Beispiel 1 erhält man durch Hydrogenolyse von 1-CBZ-amino-4S-hydroxy-5S-(4-BOC-amino-piperidinocarbonyl-amino-Pla-His-amino)-6-cyclohexyl-hexan [erhältlich durch Reaktion von 1-CBZ-amino-4S-hydroxy-5S-amino-6-cyclohexyl-hexan mit 4-BOC-amino-piperidinocarbonyl-amino-Phe-(imi-DNP-His)-OH und anschließende Abspaltung der DNP-Gruppe analog Beispiel 2] das 1-Amino-4S-hydroxy-5S-(4-BOC-amino-piperidinocarbonyl-amino-Pla-His-amino)-6-cyclohexyl-hexan.

Beispiel 4

Eine Lösung von 4,02 g N-Ethyl-N'-[4S-hydroxy-5S(H-(S-Me-Cys)-amino)-6-cyclohexyl-hexyl]-harnstoff [erhältlich aus N-Ethyl-N'-3-(2,2-dimethyl-3-BOC-4-cyclohexylmethyl-5-oxazolidinyl)-propyl-harnstoff (F. 105-106°) über N-Ethyl-N'-(4S-hydroxy-5S-amino-6-cyclohexyl-hexyl)-harnstoff und N-Ethyl-N'-(4S-hydroxy-5S-BOC-(S-Me-Cys)-amino-6-cyclohexyl-hexyl)-harnstoff (F. 144-145°)] in 60 ml Dichlormethan wird mit 1,01 g N-Methylmorpholin versetzt. Unter Rühren gibt man 3,92 g O-(4-BOC-amino-piperidinocarbonyl)-Pla-OH, 1,35 g HOBt und eine Lösung von 2,06 g DCCI in 50 ml Dichlormethan hinzu, rührt 14 Std. bei 2-6°, filtriert den ausgeschiedenen Dicyclohexylharnstoff ab und dampft das Filtrat ein. Nach üblicher Aufarbeitung erhält man N-Ethyl-N'-[4S-hydroxy-5S-(4-BOC-amino-piperidinocarbonyl-Pla-(S-Me-Cys)-amino)-6-cyclohexyl-hexyl]-harnstoff, F. 172-173°.

Analog erhält man
N-Ethyl-N'-[4S-hydroxy-5S-(pyrrolidinocarbonyl-Pla-(S-Me-Cys)-amino)-6-cyclohexyl-hexyl]-harnstoff
N-Ethyl-N'-[4S-hydroxy-5S-(piperidinocarbonyl-Pla-(S-Me-Cys)-amino)-6-cyclohexyl-hexyl]-harnstoff
N-Ethyl-N'-[4S-hydroxy-5S-(morpholinocarbonyl-Pla-(S-Me-Cys)-amino)-6-cyclohexyl-hexyl]-harnstoff, F.137° (Zers.)
N-Ethyl-N'-[4S-hydroxy-5S-(4-ethoxycarbonyl-piperidinocarbonyl-Pla-(S-Me-Cys)-amino)-6-cyclohexyl-hexyl]-harnstoff
N-Ethyl-N'-[4S-hydroxy-5S-(4-dimethylamino-piperidinocarbonyl-Pla-(S-Me-Cys)-amino)-6-cyclohexyl-hexyl]-harnstoff
N-Ethyl-N'-[4S-hydroxy-5S-(4-N-BOC-N-methylamino-piperidinocarbonyl-Pla-(S-Me-Cys)-amino)-6-cyclohexylhexyl]-harnstoff
N-Ethyl-N'-[4S-hydroxy-5S-(isobutyl-Pla-(S-Me-Cys)-amino)-6-cyclohexyl-hexyl]-harnstoff
N-Ethyl-N'-[4S-hydroxy-5S-(benzyl-Pla-(S-Me-Cys)-amino)-6-cyclohexyl-hexyl]-harnstoff
N-Ethyl-N'-[4S-hydroxy-5S-(tert.-butoxycarbonyl-Pla-(S-Me-Cys)-amino)-6-cyclohexyl-hexyl]-harnstoff
N-Ethyl-N'-[4S-hydroxy-5S-(benzyloxycarbonyl-Pla-S-Me-Cys)-amino)-6-cyclohexyl-hexyl]-harnstoff
N-[4S-Hydroxy-5S-(4-BOC-amino-piperidinocarbonyl-Pla-(S-Me-Cys)-amino)-6-cyclohexyl-hexyl]-harnstoff
N-Isopropyl-N'-[4S-hydroxy-5S-(4-BOC-amino-piperidinocarbonyl-Pla-(S-Me-Cys)-amino)-6-cyclohexyl-hexyl]-harnstoff
N-[2R-Methyl-4S-Hydroxy-5S-(4-BOC-amino-piperidinocarbonyl-Pla-(S-Me-Cys)-amino)-6-cyclohexyl-hexyl]-harnstoff
N-Ethyl-N'-[2R-methyl-4S-hydroxy-5S-(4-BOC-amino-piperidinocarbonyl-Pla-(S-Me-Cys)-amino)-6-cyclohexyl-hexyl]-harnstoff
N-Isopropyl-N'-[2R-methyl-4S-hydroxy-5S-(4-BOC-amino-piperidinocarbonyl-Pla-(S-Me-Cys)-amino)-6-cyclohexyl-hexyl]-harnstoff
N-[2S-Isopropyl-4S-hydroxy-5S-(4-BOC-amino-piperidinocarbonyl-Pla-(S-Me-Cys)-amino)-6-cyclohexyl-hexyl]-harnstoff
N-Ethyl-N'-[2S-isopropyl-4S-hydroxy-5S-(4-BOC-amino-piperidinocarbonyl-Pla-(S-Me-Cys)-amino)-6-cyclohexyl-hexyl]-harnstoff, F. 115-116° [aus N-Ethyl-N'-(2S-isopropyl-4S-hydroxy-5S-BOC-amino-6-cyclohexyl-hexyl)-harnstoff (F. 144-145°) über N-Ethyl-N'-(2S-isopropyl-4S-hydroxy-5S-BOC-(N-Me-Cys)-amino-6-cyclohexyl-hexyl]-harnstoff (F. 89-90°)]

N-Isopropyl-N'-[2S-isopropyl-4S-hydroxy-5S-(4-BOC-amino-piperidinocarbonyl-Pla-(S-Me-Cys)-amino)-6-cyclohexyl-hexyl]-harnstoff

N-[4S-Hydroxy-5S-(4-BOC-amino-piperidinocarbonyl-Pla-(S-Me-Cys)-amino)-7-methyl-octyl]-harnstoff

N-Ethyl-N'-[4S-hydroxy-5S-(4-BOC-amino-piperidinocarbonyl-Pla-(S-Me-Cys)-amino)-7-methyl-octyl]-harnstoff

N-Isopropyl-N'-[4S-hydroxy-5S-(4-BOC-amino-piperidinocarbonyl-Pla-(S-Me-Cys)-amino)-7-methyl-octyl]-harnstoff

N-Ethyl-N'-[4S-hydroxy-5S-(morpholinocarbonyl-Pla-amino-6-cyclohexyl-hexyl]-harnstoff

N-Ethyl-N'-[4S-hydroxy-5S-(4-BOC-amino-piperidinocarbonyl-Pla-amino)-6-cyclohexyl-hexyl]-harnstoff

N-Ethyl-N'-[2R-methyl-4S-hydroxy-5S-(4-BOC-amino-piperidinocarbonyl-Pla-amino)-6-cyclohexyl-hexyl]-harnstoff

N-Ethyl-N'-[2S-isopropyl-4S-hydroxy-5S-(4-BOC-amino-piperidinocarbonyl-Pla-amino)-6-cyclohexyl-hexyl]-harnstoff, F. 163-164°

N-[4S-Hydroxy-5S-(4-BOC-amino-piperidinocarbonyl-Pla-(S-Me-Cys)-amino)-6-cyclohexyl-hexyl]-propansulfonsäureamid

N-[2R-Methyl-4S-hydroxy-5S-(4-BOC-amino-piperidinocarbonyl-Pla-(S-Me-Cys)-amino)-6-cyclohexyl-hexyl]-propansulfonsäureamid

N-[2S-Isopropyl-4S-hydroxy-5S-(4-BOC-amino-piperidinocarbonyl-Pla-(S-Me-Cys)-amino)-6-cyclohexyl-hexyl]-propansulfonsäureamid

N-[4S-Hydroxy-5S-(4-BOC-amino-piperidinocarbonyl-Pla-(S-Me-Cys)-amino)-6-cyclohexyl-hexyl]-butyramid

N-[4S-Hydroxy-5S-(4-BOC-amino-piperidinocarbonyl-Pla-(S-Me-Cys)-amino)-6-cyclohexyl-hexyl]-isovaleriansäureamid

N-[4S-Hydroxy-5S-(4-BOC-amino-piperidinocarbonyl-Pla-(S-Me-Cys)-amino)-6-cyclohexyl-hexyl]-benzamid

N-[4S-Hydroxy-5S-(4-BOC-amino-piperidinocarbonyl-Pla-(S-Me-Cys)-amino)-6-cyclohexyl-hexyl]-picolinamid

N-Ethyl-N'-[4S-hydroxy-5S-(4-BOC-amino-piperidinocarbonyl-Pla-(S-Me-Cys)-amino)-6-cyclohexyl-hexyl]-thioharnstoff.

Beispiel 5

Analog Beispiel 4 erhält man aus 4-BOC-amino-piperidinocarbonyl-Pla-(S-Me-Cys)-OH und N-Ethyl-N'-(2S-isopropyl-4S-hydroxy-5S-amino-6-cyclohexyl-hexyl)-harnstoff den N-Ethyl-N'-[2S-isopropyl-4S-hydroxy-5S-(4-BOC-amino-piperidinocarbonyl-Pla-(S-Me-Cys)-amino)-6-cyclohexyl-hexyl]-harnstoff, F. 115-116°.

Analog erhält man N-Ethyl-N'-[4S-hydroxy-5S-(4-BOC-amino-piperidinocarbonyl-Pla-βAla-amino)-6-cyclohexyl-hexyl]-harnstoff

N-Ethyl-N'-[4S-hydroxy-5S-(4-BOC-amino-piperidinocarbonyl-Pla-Asn-amino)-6-cyclohexyl-hexyl]-harnstoff

N-Ethyl-N'-[4S-hydroxy-5S-(4-BOC-amino-piperidinocarbonyl-Pla-(N-Me-His)-amino)-6-cyclohexyl-hexyl]-harnstoff

N-Ethyl-N'-[4S-hydroxy-5S-(4-BOC-amino-piperidinocarbonyl-Pla-Leu-amino)-6-cyclohexyl-hexyl]-harnstoff, F. 107-108°

N-Ethyl-N'-[4S-hydroxy-5S-(4-BOC-amino-piperidinocarbonyl-Pla-Met-amino)-6-cyclohexyl-hexyl]-harnstoff

N-Ethyl-N'-[4S-hydroxy-5S-(4-BOC-amino-piperidinocarbonyl-Pla-Nle-amino)-6-cyclohexyl-hexyl]-harnstoff

N-Ethyl-N'-[4S-hydroxy-5S-(4-BOC-amino-piperidinocarbonyl-Pla-Nva-amino)-6-cyclohexyl-hexyl]-harnstoff

N-Ethyl-N'-[4S-hydroxy-5S-(4-BOC-amino-piperidinocarbonyl-Pla-(3-Pya)-amino)-6-cyclohexyl-hexyl]-harnstoff

N-Ethyl-N'-[4S-hydroxy-5S-(4-BOC-amino-piperidinocarbonyl-Pla-(2-Tia)-amino)-6-cyclohexyl-hexyl]-harnstoff

N-Ethyl-N'-[4S-hydroxy-5S-(4-BOC-amino-piperidinocarbonyl-Pla-Val-amino)-6-cyclohexyl-hexyl]-harnstoff.

Beispiel 6

Analog Beispiel 4 erhält man aus Phenoxyessigsäure (= POA-OH) und N-Propyl-N'-[4S-hydroxy-5S-(H-Pla-βAla-amino)-7-methyl-octyl]-harnstoff den N-Propyl-N'-[4S-hydroxy-5S-(POA-Pla-βAla-amino)-7-methyl-octyl]-harnstoff.

Beispiel 7

Eine Lösung von 1,25 g Cyclohexylisocyanat in 15 ml THF wird unter Rühren bei 20° zugetropft zu einer Lösung von 6,89 g 4S-Hydroxy-5S-(4-BOC-amino-piperidinocarbonyl-Pla-Leu-amino)-6-cyclohexyl-hexylamin in 60 ml THF. Man rührt noch 3 Std. bei 20°, arbeitet wie üblich auf und erhält N-Cyclohexyl-N'-[4S-hydroxy-5S-(4-BOC-amino-piperidinocarbonyl-Pla-Leu-amino)-6-cyclohexyl-hexyl]-harnstoff.

Beispiel 8

Eine Lösung von 1 g N-Ethyl-N'-[4S-hydroxy-5S-(4-BOC-amino-piperidinocarbonyl-Pla-His-amino)-6-cyclohexyl-hexyl]-harnstoff in 20 ml Dichlormethan und 20 ml Trifluoressigsäure wird 1 Std. bei 20° gerührt und dann eingedampft. Man erhält N-Ethyl-N'-[4S-hydroxy-5S-(4-amino-piperidinocarbonyl-Pla-His-amino)-6-cyclohexyl-hexyl]-harnstoff, Bis-TFA, F. 141° (Zers.).

Analog erhält man durch Spaltung der entsprechenden BOC-Derivate:

N-[4S-Hydroxy-5S-(4-amino-piperidinocarbonyl-Pla-His-amino)-6-cyclohexyl-hexyl]-harnstoff

N-Isopropyl-N'-[4S-hydroxy-5S-(4-amino-piperidinocarbonyl-Pla-His-amino)-6-cyclohexyl-hexyl]-harnstoff

N-[2R-Methyl-4S-hydroxy-5S-(4-amino-piperidinocarbonyl-Pla-His-amino)-6-cyclohexyl-hexyl]-harnstoff

N-Ethyl-N'-[2R-methyl-4S-hydroxy-5S-(4-amino-piperidino-carbonyl-Pla-His-amino)-6-cyclohexyl-hexyl]-harnstoff

N-Isopropyl-N'-[2R-methyl-4S-hydroxy-5S-(4-amino-piperidinocarbonyl-Pla-His-amino)-6-cyclohexyl-hexyl]-harnstoff, Bis-TFA, F. 141°

N-[2S-Isopropyl-4S-hydroxy-5S-(4-amino-piperidinocarbonyl-Pla-His-amino)-6-cyclohexyl-hexyl]-harnstoff

N-Ethyl-N'-[2S-isopropyl-4S-hydroxy-5S-(4-amino-piperidinocarbonyl-Pla-His-amino)-6-cyclohexyl-hexyl]-harnstoff, Bis-TFA, F. 154-155°

N-Isopropyl-N'-[2S-isopropyl-4S-hydroxy-5S-(4-amino-piperidinocarbonyl-Pla-His-amino)-6-cyclohexyl-hexyl]-harnstoff

N-Ethyl-N'-[4S-hydroxy-5S-(4-amino-piperidinocarbonyl-Pla-βAla-amino)-6-cyclohexyl-hexyl]-harnstoff

N-Ethyl-N'-[4S-hydroxy-5S-(4-amino-piperidinocarbonyl-Pla-Asn-amino)-6-cyclohexyl-hexyl]-harnstoff

N-Ethyl-N'-[4S-hydroxy-5S-(4-amino-piperidinocarbonyl-Pla-(N-Me-His)-amino)-6-cyclohexyl-hexyl]-harnstoff

N-Ethyl-N'-[4S-hydroxy-5S-(4-amino-piperidinocarbonyl-Pla-Leu-amino)-6-cyclohexyl-hexyl]-harnstoff, TFA, F. 152-153°

N-Ethyl-N'-[4S-hydroxy-5S-(4-amino-piperidinocarbonyl-Pla-Met-amino)-6-cyclohexyl-hexyl]-harnstoff

N-Ethyl-N'-[4S-hydroxy-5S-(4-amino-piperidinocarbonyl-Pla-Nle-amino)-6-cyclohexyl-hexyl]-harnstoff

N-Ethyl-N'-[4S-hydroxy-5S-(4-amino-piperidinocarbonyl-Pla-Nva-amino)-6-cyclohexyl-hexyl]-harnstoff

N-Ethyl-N'-[4S-hydroxy-5S-(4-amino-piperidinocarbonyl-Pla-(3-Pya)-amino)-6-cyclohexyl-hexyl]-harnstoff

N-Ethyl-N'-[4S-hydroxy-5S-(4-amino-piperidinocarbonyl-Pla-(2-Tia)-amino)-6-cyclohexyl-hexyl]-harnstoff

N-Ethyl-N'-[4S-hydroxy-5S-(4-amino-piperidinocarbonyl-Pla-Val-amino)-6-cyclohexyl-hexyl]-harnstoff

N-[4S-Hydroxy-5S-(4-amino-piperidinocarbonyl-Pla-(S-Me-Cys)-amino)-6-cyclohexyl-hexyl]-harnstoff

N-Ethyl-N'-[4S-hydroxy-5S-(4-amino-piperidinocarbonyl-Pla-(S-Me-Cys)-amino)-6-cyclohexyl-hexyl]-harnstoff, TFA, F. 113-114°

N-Isopropyl-N'-[4S-hydroxy-5S-(4-amino-piperidinocarbonyl-Pla-(S-Me-Cys)-amino)-6-cyclohexyl-hexyl]-harnstoff

N-[2R-Methyl-4S-hydroxy-5S-(4-amino-piperidinocarbonyl-Pla-(S-Me-Cys)-amino)-6-cyclohexyl-hexyl]-harnstoff

N-Ethyl-N'-[2R-Methyl-4S-hydroxy-5S-(4-amino-piperidino-carbonyl-Pla-(S-Me-Cys)-amino)-6-cyclohexyl-hexyl]-harnstoff

N-Isopropyl-N'-[2R-Methyl-4S-hydroxy-5S-(4-amino-piperidinocarbonyl-Pla-(S-Me-Cys)-amino)-6-cyclohexyl-hexyl]-harnstoff

N-[2S-Isopropyl-4S-hydroxy-5S-(4-amino-piperidinocarbonyl-Pla-(S-Me-Cys)-amino)-6-cyclohexyl-hexyl]-harnstoff

N-Ethyl-N'-[2S-Isopropyl-4S-hydroxy-5S-(4-amino-piperidinocarbonyl-Pla-(S-Me-Cys)-amino)-6-cyclohexyl-hexyl]-harnstoff, TFA, F. 123-124°

N-Isopropyl-N'-[2S-Isopropyl-4S-hydroxy-5S-(4-amino-piperidinocarbonyl-Pla-(S-Me-Cys)-amino)-6-cyclohexyl-hexyl]-harnstoff

N-[4S-Hydroxy-5S-(4-amino-piperidinocarbonyl-Pla-(S-Me-Cys)-amino)-7-methyl-octyl]-harnstoff

N-Ethyl-N'-[4S-hydroxy-5S-(4-amino-piperidinocarbonyl-Pla-(S-Me-Cys)-amino)-7-methyl-octyl]-harnstoff

N-Isopropyl-N'-[4S-hydroxy-5S-(4-amino-piperidinocarbonyl-Pla-(S-Me-Cys)-amino)-7-methyl-octyl]-harnstoff

N-Ethyl-N'-[4S-hydroxy-5S-(4-amino-piperidinocarbonyl-Pla-amino)-6-cyclohexyl-hexyl]-harnstoff

N-Ethyl-N'-[2R-methyl-4S-hydroxy-5S-(4-amino-piperidinocarbonyl-Pla-amino)-6-cyclohexyl-hexyl]-harnstoff

N-Ethyl-N'-[2S-isopropyl-4S-hydroxy-5S-(4-amino-piperidinocarbonyl-Pla-amino)-6-cyclohexyl-hexyl]-harnstoff, TFA, F. 135-136°

N-Ethyl-N'-[4S-hydroxy-5S-(4-methylamino-piperidinocarbonyl-Pla-(S-Me-Cys)-amino)-6-cyclohexyl-hexyl]-harnstoff

N-Ethyl-N'-[4S-hydroxy-5S(2S-(4-amino-piperidinocarbonyloxy)-4-methylbutyryl-His-amino)-6-cyclohexyl-hexyl]-harnstoff

N-Ethyl-N'-[2S-isopropyl-4S-hydroxy-5S-(2S-(4-amino-piperidinocarbonyloxy-4-methylbutyryl-His-amino)-6-cyclohexyl-hexyl]-harnstoff N-Ethyl-N'-[4S-hydroxy-5S-(2S-(4-amino-piperidinocarbonyloxy)-hexanoyl-His-amino)-6-cyclohexyl-hexyl]-harnstoff

N-Ethyl-N'-[2S-isopropyl-4S-hydroxy-5S-(2S-(4-amino-piperidinocarbonyloxy)-hexanoyl-His-amino)-6-cyclohexyl-hexyl]-harnstoff

N-Ethyl-N'-[4S-hydroxy-5S-(2S-(4-amino-piperidinocarbonyloxy)-3-cyclohexyl-propionyl-His-amino)-6-cyclohexyl-hexyl]-harnstoff

N-Ethyl-N'-[2S-isopropyl-4S-hydroxy-5S-(2S-(4-amino-piperidinocarbonyloxy)-3-cyclohexyl-propionyl-His-amino)-6-cyclohexyl-hexyl]-harnstoff

N-Ethyl-N'-[4S-hydroxy-5S-(2S-(4-amino-piperidinocarbonyloxy)-3-p-methoxyphenyl-propionyl-His-amino)-6-cyclohexyl-hexyl]-harnstoff

N-Ethyl-N'-[2S-isopropyl-4S-hydroxy-5S-(2S-(4-amino-piperidinocarbonyloxy)-3-p-methoxyphenyl-propionyl-His-amino)-6-cyclohexyl-hexyl]-harnstoff

N-Ethyl-N'-[4S-hydroxy-5S-(2S-(4-amino-piperidinocarbonyloxy)-3-(2-thienyl)-propionyl-His-amino)-6-cyclohexyl-hexyl]-harnstoff

N-Ethyl-N'-[2S-isopropyl-4S-hydroxy-5S-(2S-(4-amino-piperidinocarbonyloxy)-3-(2-thienyl)-propionyl-His-amino)-6-cyclohexyl-hexyl]-harnstoff

N-[4S-Hydroxy-5S-(4-amino-piperidinocarbonyl-Pla-(His-amino)-6-cyclohexyl-hexyl]-propansulfonsäureamid

N-[2R-Methyl-4S-hydroxy-5S-(4-amino-piperidinocarbonyl-Pla-His-amino)-6-cyclohexyl-hexyl]-propansulfonsäureamid, Bis-TFA, F. 136° (Zers.)

N-[2S-isopropyl-4S-hydroxy-5S-(4-amino-piperidinocarbonyl-Pla-His-amino)-6-cyclohexyl-hexyl]-propansulfonsäureamid, Bis-TFA, F. 141-143°

N-[4S-Hydroxy-5S-(4-amino-piperidinocarbonyl-Pla-(S-Me-Cys)-amino)-6-cyclohexyl-hexyl]-propansulfonsäureamid

N-[2R-Methyl-4S-hydroxy-5S-(4-amino-piperidinocarbonyl-Pla-(S-Me-Cys)-amino)-6-cyclohexyl-hexyl]-propansulfonsäureamid

N-[2S-Isopropyl-4S-hydroxy-5S-(4-amino-piperidinocarbonyl-Pla-(S-Me-Cys)-amino)-6-cyclohexyl-hexyl]-propansulfonsäureamid

N-[4S-Hydroxy-5S-(4-amino-piperidinocarbonyl-Pla-(His-amino)-7-methyl-octyl]-propansulfonsäureamid

N-[2R-Methyl-4S-hydroxy-5S-(4-amino-piperidinocarbonyl-Pla-(His-amino)-7-methyl-octyl]-propansulfonsäureamid

N-[2S-Isopropyl-4S-hydroxy-5S-(4-amino-piperidinocarbonyl-Pla-(His-amino)-7-methyl-octyl]-propansulfonsäureamid

N-[4S-Hydroxy-5S-(4-amino-piperidinocarbonyl-Pla-(His-amino)-6-phenyl-hexyl]-propansulfonsäureamid

N-[2R-Methyl-4S-hydroxy-5S-(4-amino-piperidinocarbonyl-Pla-(His-amino)-6-methyl-hexyl]-propansulfonsäureamid

N-[2S-Isopropyl-4S-hydroxy-5S-(4-amino-piperidinocarbonyl-Pla-(His-amino)-6-phenyl-hexyl]-propansulfonsäureamid

N-[4S-Hydroxy-5S-(4-amino-piperidinocarbonyl-Pla-(His-amino)-6-cyclohexyl-hexyl]-isovaleriansäureamid, Bis-TFA, F. 122-123°

N-[4S-Hydroxy-5S-(4-amino-piperidinocarbonyl-Pla-(S-Me-Cys)-amino)-6-cyclohexyl-hexyl]-butyramid

N-[4S-Hydroxy-5S-(4-amino-piperidinocarbonyl-Pla-(S-Me-Cys)-amino)-6-cyclohexyl-hexyl]-isovaleriansäureamid

N-[4S-Hydroxy-5S-(4-amino-piperidinocarbonyl-Pla-(S-Me-Cys)-amino)-6-cyclohexyl-hexyl]-benzamid

N-[4S-Hydroxy-5S-(4-amino-piperidinocarbonyl-Pla-(S-Me-Cys)-amino)-6-cyclohexyl-hexyl]-picolinamid

N-Ethyl-N'-[4S-hydroxy-5S-(4-amino-piperidinocarbonyl-Pla-(S-Me-Cys)-amino)-6-cyclohexyl-hexyl]-thioharnstoff.

**Beispiel 9**

Ein Gemisch von 1 g N-Ethyl-N'-[4S-hydroxy-5S-(4-ethoxycarbonyl-piperidinocarbonyl-Pla-(S-Me-Cys)-amino)-6-cyclohexyl-hexyl]-harnstoff, 50 ml Dioxan und 20 ml 2 n wässerige NaOH-Lösung wird 3 Std. bei 20° gerührt. Nach üblicher Aufarbeitung erhält man N-Ethyl-N'-[4S-hydroxy-5S-(4-carboxy-piperidinocarbonyl-Pla-(S-Me-Cys)-amino)-6-cyclohexyl-hexyl]-harnstoff.

**Beispiel 10**

Eine Lösung von 6,89 g 4S-Hydroxy-5S-(4-BOC-amino-piperidinocarbonyl-Pla-Leu-amino)-6-cyclohexyl-hexylamin in 250 ml THF wird mit 1,01 g Triethylamin und dann unter Rühren bei 0° tropfenweise mit einer Lösung von 1,15 g Methansulfonylchlorid in 10 ml THF versetzt. Man rührt noch 1 Std., wobei man die Temperatur auf 20° steigen läßt, arbeitet wie üblich auf und erhält N-[4S-Hydroxy-5S-(4-BOC-amino-piperidinocarbonyl-Pla-Leu-amino)-6-cyclohexyl-hexyl]-methansulfonsäureamid.

**Beispiel 11**

Analog Beispiel 1 erhält man aus N-Ethyl-N'-[4S-hydroxy-5S-(4-CBZ-amino-piperidinocarbonyl-Pla-(S-Me-Cys)-amino)-6-cyclohexyl-hexyl]-harnstoff den N-Ethyl-N'-[4S-hydroxy-5S-(4-amino-piperidinocarbonyl-Pla-(S-Me-Cys)-amino)-6-cyclohexyl-hexyl]-harnstoff; TFA, F. 123-124°.

**Beispiel 12**

a) Analog Beispiel 4 erhält man aus 4-Dimethylaminobutyryl-Pla-Gly-OH und N-(2S-Isopropyl-4-oxo-5S-amino-6-cyclohexyl-hexyl)-propansulfonsäureamid das N-[2S-Isopropyl-4-oxo-5S-(4-dimethylaminobutyryl-Pla-Gly-amino)-6-cyclohexyl-hexyl]-propansulfonsäureamid.

b) Eine Lösung von 1 g des vorstehenden Keto-amids in 25 ml CH₃OH wird an 0,1 g 10%ig. Pd-C bei 20° und 1 bar bis zum Stillstand der H₂-Aufnahme hydriert. Hach Filtrieren und Eindampfen erhält man ein Gemisch von N-[2S-Isopropyl-4R- und -4S-hydroxy-5S-(4-dmethylaminobutyryl-Pla-Gly-amino)-6-cyclohexyl-hexyl]-propansulfonsäureamid.

Beispiel 13

Eine Lösung von 627 mg des nach Beispiel 12a) erhältlichen Keto-amids und 1,43 g Na₂CO₃ . 10 H₂O in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert. Man filtriert den Katalysator ab, dampft das Filtrat ein, trennt das erhaltene Gemisch an Kieselgel und erhält N-[2S-Isopropyl-4S-amino-5S-(4-dimethylaminobutyryl-Pla-Gly-amino)-6-cyclohexyl-hexyl]-propansulfonsäureamid; daneben erhält man das 4R-Amino-Epimere.

Beispiel 14

Analog Beispiel 1 erhält man durch Hydrogenolyse von N-Ethyl-N'-[4S-hydroxy-5S-(morpholinocarbonyl-Pla-(imi-BOM-His)-amino)-6-cyclohexyl-hexyl]-harnstoff den N-Ethyl-N'-[4S-hydroxy-5S-(morpholinocarbonyl-Pla-(His-amino)-6-cyclohexyl-hexyl]-harnstoff, Hydrochlorid, F. 126-127°.

Beispiel 15

Analog Beispiel 8 erhält man durch Spaltung von N-Ethyl-N'-[4S-hydroxy-5S-(BOC-Pla-(S-Me-Cys)-amino)-6-cyclohexyl-hexyl]-harnstoff den N-Ethyl-N'-[4S-hydroxy-5S-(H-Pla-(S-Me-Cys)-amino)-6-cyclohexyl-hexyl]-harnstoff, F. 125-126°.

Die nachfolgenden Beispiele betreffen pharmazeutische Zubereitungen.

Beispiel A: Tabletten

Ein Gemisch von 1 kg N-[2S-Isopropyl-4S-hydroxy-5S-(4-aminopiperidinocarbonyl-Pla-His-amino)-6-cyclohexylhexyl]-propansulfonsäureamid-bis-trifluoracetat, 4 kg Lactose, 1,2 kg Maisstärke, 200 g Talk und 100 g Magnesiumstearat wird in üblicher Weise zu Tabletten verpreßt, derart daß jede Tablette 100 mg Wirkstoff enthält.

Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

Beispiel C: Kapseln

500 g N-Ethyl-N'-[2S-isopropyl-4S-hydroxy-5S-(4-aminopiperidinocarbonyl-Pla-His-amino)-6-cyclohexyl-hexyl]-harnstoff-bis-trifluoracetat werden in üblicher Weise in Hartgelatinekapseln gefüllt, so daß jede Kapsel 500 mg Wirkstoff enthält.

Beispiel D: Injektionsgläser

Eine Lösung von 100 g N-Ethyl-N'-[4S-hydroxy-5S-(4-aminopiperidinocarbonyl-Pla-(S-Me-Cys)-amino)-6-cyclohexyl-hexyl]-harnstoff-trifluoracetat in 4 l zweifach destilliertem Wasser wird mit 2n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

Beispiel E: Suppositorien

Man schmilzt ein Gemisch von 50 g N-Ethyl-N'-[4S-hydroxy-5S-(4-amino-piperidinocarbonyl-Pla-(S-Me-Cys)-amino)-6-cyclohexyl-hexyl]-harnstoff-trifluoracetat mit 10 g Sojalecithin und 140 g Kakaobutter, gießt in Formen und läßt erkalten. Jedes Suppositorium enthält 250 mg Wirkstoff.

**Patentansprüche**

1. Glykolsäurederivate der Formel I

X-O-CR¹R²-CO-Y-NR³-CHR⁴-CR⁵-CH₂-CR⁶R⁷-Z    I

worin

X

$R^8$, $R^8$-O-$C_mH_{2m}$-CO-, $R^8$-$C_mH_{2m}$-O-CO-, $R^8$-$C_mH_{2m}$-CO-, $R^8$-SO₂-, $R^9R^{10}$N-$C_mH_{2m}$-CO-, $R^{11}$-NH-C(=NH)-NH-$C_mH_{2m}$-CO-, $R^9$OOC-$C_mH_{2m}$-CO-, $R^9O_3$S-$C_mH_{2m}$-CO-, $R^9$-O-(CH₂CH₂O)ₙ-$C_mH_{2m}$-CO-oder An⁻N⁺A₃-$C_mH_{2m}$-CO-,

Y

0 oder 1 Aminosäurerest ausgewählt aus der Gruppe bestehend aus Abu, Ada, Ala, βAla, Arg, Asn, Asp, Bia, Cal, Cys, S-A-Cys, Dab, Gln, Glu, Gly, His, N(im)-A-His, Hph, Ile, Leu, tert.-Leu, Lys, Mal, Met, αNal, βNal, Nbg, Nle, Nva, Orn, Phe, Pia, Pro, Pya, Ser, Thr, Tia, Tic, Trp, Tyr und Val,

Z

-CN, -CH$_2$-NR$^{12}$R$^{13}$, -CH$_2$-NR$^{12}$-SO$_2$R$^{14}$, -CH$_2$-NR$^{12}$-COR$^{14}$, -CH$_2$-NR$^{12}$-CO-NH-R$^{14}$, oder -CH$_2$-NR$^{12}$-CS-NH-R$^{14}$

R$^1$, R$^3$, R$^6$, R$^7$,R$^9$, R$^{10}$, R$^{12}$ und R$^{13}$

jeweils H oder A,

R$^2$, R$^4$, R$^8$ und R$^{14}$

jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R$^5$

(H, OH), (H, NH$_2$) oder = O,

R$^{11}$

H, A oder CN,

R$^9$R$^{10}$N und NR$^{12}$R$^{13}$

auch jeweils eine unsubstituierte oder eine durch A, OH, NH$_2$, NHA, NA$_2$, NHAc, NH-CO-C$_x$H$_{2x}$-O-R$^{15}$ , NH-CO-O-C$_x$H$_{2x}$-R$^{15}$, Hydroxyalkyl, COOH, COOA, CONH$_2$, Aminoalkyl, HAN-alkyl, A$_2$N-alkyl, A$_3$N$^\oplus$ alkyl An$^\ominus$, NH-CO-NH$_2$, NH-CO-NHA, Guanidinyl oder Guanidinyl-alkyl substituierte Pyrrolidino-, Piperidino-, Morpholino-oder Piperazinogruppe,

R$^{15}$

A oder Ar-alkyl,

m, n und x

jeweils 0, 1, 2, 3, 4, 5, 6, 7, 8, 9 oder 10,

Ar

unsubstituiertes oder ein- oder mehrfach durch A, OA, Hal, CF$_3$, OH, NO$_2$, Hydroxyalkyl, NH$_2$, NHA, NA$_2$, NHAc, NH-SO$_2$-A, SA, SO-A, SO$_2$-A, SO$_2$NH$_2$, SO$_2$NHA, COOH, COOA, CONH$_2$, CN, Amino-alkyl, HAN-alkyl, A$_2$N-alkyl, A$_3$N$^\oplus$-alkyl An$^\ominus$ und/oder Guanidinyl-alkyl substituiertes Phenyl oder unsubstituiertes Naphthyl,

Het

einen gesättigten oder ungesättigten 5-oder 6-gliedrigen heterocyclischen Rest mit 1-4 N-, O- und/oder S-Atomen, der mit einem Benzolring kondensiert und/oder ein-oder mehrfach durch A, OA, Hal, CF$_3$, OH, NO$_2$, Carbonylsauerstoff, NH$_2$, NHA, NA$_2$, NHAc, NH-COOA, NHCOOAr, NHCOOCH$_2$Ar, NH-SO$_2$-A, SA, SO-A, SO$_2$-A, SO$_2$NH$_2$, SO$_2$NHA, COOH, COOA, CONH$_2$, CN, Ar, Ar-alkyl, Ar-alkenyl, Hydroxy-alkyl, Aminoalkyl, HAN-alkyl, A$_2$N-alkyl und/oder A$_3$N$^\oplus$-alkyl An$^\ominus$ substituiert sein kann und/oder dessen N-und/oder S-Heteroatome auch oxydiert sein können,

Hal

F, Cl, Br oder J,

Ac

A-CO-, Ar-CO-, Ar-alkyl-CO- oder A-NH-CO- ,

An$^\ominus$

ein Anion, das auch fehlen kann, wenn stattdessen eine in der Verbindung der Formel I enthaltene Carboxygruppe in Form eines Carboxylatanions vorliegt,

-alkyl-

eine Alkylengruppe mit 1-8 C-Atomen und

A

Alkyl mit 1-8 C-Atomen bedeuten, worin ferner an Stelle einer oder mehrerer -NH-CO-Gruppen auch eine oder mehrere -NA-CO-Gruppen stehen können, sowie deren Salze.

2.

    a)             N-[2-Isopropyl-4-hydroxy-5-(4-aminopiperidinocarbonyl-Pla-His-amino)-6-cyclohexyl-hexyl]-propansulfonsäureamid;

    b)             N-Ethyl-N'-[4-hydroxy-5-(4-aminopiperidinocarbonyl-Pla-His-amino)-6-cyclohexyl-hexyl]-harnstoff;

    c)             N-Ethyl-N'-[4-hydroxy-5-(4-aminopiperidinocarbonyl-Pla- (S-Me-Cys)-amino)-6-cyclohexyl-hexyl]-harnstoff.

3.     Verfahren zur Herstellung eines Glykolsäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

X-G$^1$-OH     II

worin

G$^1$     (a) fehlt,

        (b) -O-CR$^1$R$^2$-CO-,

        (c) -O-CR$^1$R$^2$-CO-Y- bedeutet

oder eines ihrer reaktionsfähigen Derivate mit einer Verbindung der Formel III

H-G$^2$-NR$^3$-CHR$^4$-CR$^5$-CH$_2$-CR$^6$R$^7$-Z     III

worin

G$^2$     (a) -O-CR$^1$R$^2$-CO-Y-,

        (b) -Y- bedeutet,

        (c) fehlt,

umsetzt, und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt

33        **EP 0 446 751 A1**        34

und/oder eine freie Aminogruppe durch Behandeln mit einem acylierenden Mittel acyliert und/oder zur Herstellung einer Verbindung der Formel I, $R^5$ = (H, OH) oder (H, $NH_2$), ein Aminoketosäurederivat der Formel I, $R^5$ = O, reduziert oder reduktiv aminiert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.

# EUROPÄISCHER TEILRECHERCHENBERICHT,

Europäisches Patentamt

der nach Regel 45 des Europäischen Patentübereinkommens für das weitere Verfahren als
europäischer Recherchenbericht gilt

## EINSCHLÄGIGE DOKUMENTE

EP 91103213.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP - A2 - 0 300 189 (FUJISAWA) <br> * Ansprüche 1,13-15 * <br> -- | 1,5,6 | C 07 D 401/12 <br> C 07 D 409/12 <br> C 07 D 211/56 <br> C 07 D 233/64 |
| A | EP - A2 - 0 310 918 (HOFFMANN) <br> * Ansprüche 1,16-20 * <br> -- | 1,5,6 | C 07 D 265/28 <br> C 07 C 255/26 <br> C 07 C 255/24 <br> C 07 C 237/04 |
| A | EP - A2 - 0 184 550 (CIBA-GEIGY) <br> * Zusammenfassung; Anspruch 1 * <br> -- | 1,6 | C 07 C 237/22 <br> C 07 C 227/40 <br> C 07 C 317/28 <br> A 61 K 31/415 <br> A 61 K 31/445 |
| A | EP - A2 - 0 258 183 (CIBA-GEIGY) <br> * Zusammenfassung; Formel I * <br> -- | 1,6 | |
| A | EP - A2 - 0 156 322 (MERCH) <br> * Ansprüche 1,15; Formel I * <br> -- | 1,5,6 | |
| A | WO - A1 - 88/03 022 (PFIZER) <br> * Ansprüche 1,21,22 * | 1,5,6 | |

|  |
|---|
| RECHERCHIERTE SACHGEBIETE (Int. Cl.5) |
| C 07 D 401/00 <br> C 07 D 409/00 <br> C 07 D 211/00 <br> C 07 D 233/00 <br> C 07 D 265/00 <br> C 07 C 255/00 <br> C 07 C 237/00 <br> C 07 C 227/00 <br> C 07 C 317/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: *1-6*

Unvollständig recherchierte Patentansprüche: *—*

Nicht recherchierte Patentansprüche: *7*

Grund für die Beschränkung der Recherche:

*Art 52(4) EPÜ*    *Verfahren zur therapeutischen*
*Behandlung des menschlichen*
*und tierischen Körpers*

| Recherchenort | Abschlußdatum der Recherche | Prüfer |
|---|---|---|
| WIEN | 02-05-1991 | HAMMER |

Europäisches
Patentamt

**EUROPÄISCHER TEILRECHERCHENBERICHT**

Nummer der Anmeldung
−2−
EP 91103213.4

| EINSCHLÄGIGE DOKUMENTE | | | KLASSIFIKATION DER ANMELDUNG (Int. Cl.⁵) |
|---|---|---|---|
| Kategorie | Kennzeichnung des Dokuments mit Angabe, soweit erforderlich, der maßgeblichen Teile | betrifft Anspruch | |
| | -- | | |
| D,A | EP − A2 − 0 249 096 (MERCH) <br> * Ansprüche 1,5-7 * <br> -- | 1,5,6 | |
| A | CHEMICAL ABSTRACTS, Band 109, Nr. 15, 10. Oktober 1988, Columbus, Ohio, USA IIZUKA, KINJI et al. "New human renin inhibitory peptides: angiotensinogen transition-state analogs containing novel Leu-Val replacement and a retro-inverso amide bond" Seite 1, Spalte 1, Zusammenfassung-Nr. 121 816y & Chem. Pharm. Bull. 1988, 36(6),2278-81 <br> -- | 1,5,6 | RECHERCHIERTE SACHGEBIETE (Int. Cl.⁵) |
| A | CHEMICAL ABSTRACTS, Band 110, Nr. 25, 19. Juni 1989, Columbus, Ohio, USA HANSON, GUNNAR J. et al. "A new class of orally active glycol renin inhibitors containing phenyllactic acid at P3" Seite 16, Spalte 2, Zusammenfassung-Nr. 225 010t & Biochem. Biophys. Res. Commun. 1989,160(1),1-5 <br> -- | 1,5,6 | |
| P,X | CHEMICAL ABSTRACTS, Band 114, Nr. 9, 4. März 1991, Columbus, Ohio, USA CUMIN, F. et al. "Pharmacokinetics and tissue distribution of the renin inhibitor N-(2-(R)-benzyl-3-tert-butyl-sulfonyl-propionyl)-His-ChacVal-N-butylamine in marmosets" Seite 9, Spalte 2, Zusammenfassung-Nr. 74 652h & Drug Metab. Dispos. 1990, | 1,5,6 | |

| EINSCHLÄGIGE DOKUMENTE | | | KLASSIFIKATION DER<br>ANMELDUNG (Int. Cl.45 |
|---|---|---|---|
| Kategorie | Kennzeichnung des Dokuments mit Angabe, soweit erforderlich, der<br>maßgeblichen Teile | betrifft<br>Anspruch | |
| | 18(6),831−5<br>−− | | |
| A | CHEMICAL ABSTRACTS, Band 107,<br>Nr. 23, 7. Dezember 1987,<br>Columbus, Ohio, USA<br>KEMPF, DALE J. et al.<br>"Renin inhibitors based on<br>novel dipeptide analogs. In-<br>corporation of the dehydro-<br>hydroxyethylene isostere at<br>the scissile bond"<br>Seite 638, Spalte 1, Zu-<br>sammenfassung-Nr. 218 063x<br>    & J. Med. Chem. 1987,30(11),<br>    1978-83<br>−− | 1,5,6 | |
| A | CHEMICAL ABSTRACTS, Band 109,<br>Nr. 11, 12. September 1988,<br>Columbus, Ohio, USA<br>BUEHLMAYER, PETER et al.<br>"Synthesis and biological<br>activity of some transition-<br>state inhibitors of human<br>renin"<br>Seite 772, Spalte 2, Zu-<br>sammenfassung-Nr. 93 588e<br>    & J. Med. Chem. 1988,31(9),<br>    1839-46<br>−−−− | 1,5,6 | RECHERCHIERTE<br>SACHGEBIETE (Int. Cl.45 |

EPA Form 1505.3   06.78